# EUROPEAN PATENT SPECIFICATION

(11) **EP 2 512 329 B1**
(45) Date of publication and mention of the grant of the patent: **12.07.2017**
(21) Application number: 10795866.2
(22) Date of filing: 10.12.2010
(51) Int. Cl.: A61B 5/0215, A61B 5/026, A61M 25/00, A61M 25/10, A61B 17/12, A61B 17/00, A61B 18/00, A61B 18/14, A61B 18/02

(54) **VEIN OCCLUSION DEVICES FOR CATHETER-BASED ABLATION**
VENENVERSCHLUSSVORRICHTUNGEN FÜR KATHETERBASIERTE ABLATION
DISPOSITIFS D'OCCLUSION VEINEUSE POUR ABLATION PAR CATHÉTER

(30) Priority: 11.12.2009 US 635821
(43) Date of publication of application: 24.10.2012
(73) Proprietor: MEDTRONIC CRYOCATH LP, Toronto, ON M5L 1B9 (CA)
(72) Inventor: PAGEARD, Jean-Luc, Montreal Québec H3C 6N1 (CA); MIHALIK, Teresa, A., Montreal Québec H3X 3P8 (CA)
(74) Representative: Casey, Lindsay Joseph
(86) International application number: PCT/US2010/059795
(87) International publication number: WO 2011/072186

(56) References cited:
- WO-A1-2008/064387
- WO-A2-2008/091570
- US-A- 3 798 967
- US-A1- 2003 176 816
- US-A1- 2005 148 997
- US-A1- 2005 215 989
- US-A1- 2006 259 023
- US-A1- 2007 083 194
- US-A1- 2008 228 141
- US-A1- 2009 326 526

## Description

### TECHNICAL FIELD

The present disclosure relates to catheter-based methods, systems, devices for occlusion, and in particular, utilizing measurements of one or more physiological parameters to guide an ablation treatment of cardiac arrhythmias.

### BACKGROUND

Catheter based devices are employed in various medical and surgical applications because they are relatively non-invasive and allow for precise treatment of localized tissues that are otherwise inaccessible. Catheters may be easily inserted and navigated through the blood vessels and arteries, allowing non-invasive access to areas of the body with relatively little trauma. Recently, catheter-based systems have been developed for implementation in tissue ablation for treatment of cardiac arrhythmias such as atrial fibrillation, supra ventricular tachycardia, atrial tachycardia, ventricular tachycardia, ventricular fibrillation, and the like. One such implementation involves the use of fluids with low operating temperatures, or cryogens, to selectively freeze, or "cold-treat", targeted tissues within the body.

The cryogenic treatment involves cooling a portion of the catheter to a very low temperature through the use of the cryogenic fluid flowing through the catheter. A cryogenic device uses the energy transfer derived from thermodynamic changes occurring in the flow of a cryogen therethrough to create a net transfer of heat flow from the target tissue to the device, through conductive and convective heat transfer between the cryogen and target tissue.

Structurally, cooling can be achieved through injection of high-pressure coolant into a lumen of the catheter. Upon injection, the refrigerant undergoes two primary thermodynamic changes: (i) expanding to low pressure and temperature through positive Joule-Thomson throttling, and (ii) undergoing a phase change from liquid to vapor, thereby absorbing heat of vaporization. The resultant flow of low temperature refrigerant through the device acts to absorb heat from the target tissue and thereby cool the tissue to the desired temperature.

Once refrigerant is injected into the lumen, it may be expanded inside of an expandable chamber, which is positioned proximal to the target tissue. In embodiments, the expandable chamber may also be thermally conductive. Devices with an expandable chamber, such as a balloon, may be employed. Such a device is disclosed in U.S. Patent 7,300,433, Lane et al.. In such a device, refrigerant is supplied through a catheter lumen into an expandable balloon coupled to such catheter, wherein the refrigerant acts to both: (i) expand the balloon near the target tissue for the purpose of positioning the balloon, and (ii) cool the target tissue proximal to the balloon to cold-treat adjacent tissue.

The expandable chamber may also serve a second function; blocking the flow of blood through the desired treatment site (occlusion). The catheter is typically of a relatively small diameter and long body, generally determined, by the diameter and length of the vascular pathways leading to the ablation site. The coolant in the catheter is highly susceptible to conductive warming effects due to the relative proximity of the catheter (and coolant) to the body tissue and blood. Furthermore, the rate of cooling is limited by the ability to circulate a sufficient mass flow of coolant through the catheter. Yet there is a requirement that the coolant itself be at a sufficiently low temperature, in some cases below freezing, at the location of the ablation.

Radio frequency (RF) catheter ablation is another common implementation of the catheter-based treatment. Arrays of ablation elements including but not limited to geometrically-adjustable electrode arrays, may be configured in a wide variety of ways and patterns on the catheter as disclosed for example in U.S. Application 2007/083194 by Kunis et al.. Such elements may be coupled to the expandable chamber or other portions of the catheter. RF catheter ablation includes a preliminary step of conventional electrocardiographic mapping followed by the creation of one or more ablated regions (lesions) in the cardiac tissue using RF energy. RF energy applied by the catheter elevates the temperature of the tissue for therapeutic treatment of an arrhythmia. The effectiveness of the RF energy may be limited by the flow of blood; the rapid blood flow carries away the generated heat and causes cooling of the ablating electrodes and/or tissue.

Therefore, blocking the flow of blood using the expandable chamber allows more effective cooling or heating (depending on the treatment method) which facilitates the treatment process and may reduce the treatment period. Effective contact to achieve occlusion may require moving, positioning, anchoring and other mechanisms for locating and stabilizing the conformation of the expandable chamber of the catheter. Moreover, slight changes in orientation may greatly alter the characteristics of the catheter, so that even when the changes are predictable or measurable, it may become necessary to provide positioning mechanisms of high stability or accuracy to assure adequate treatment at the designated sites. Furthermore, one must assure that the ablation activity is effective at the target tissue.

Known techniques for visualizing the contact between the expandable chamber and the target tissue include the use of radiographically opaque contrast medium to enable radiographic-mapping of the target tissue during application and operation of the catheter. Such an imaging technique may not be desirable due to the use of contrast medium and its interaction with the patient tissue. Additionally, it may be desirable to eliminate or minimize the exposure of both patient and clinician to the radiographic-mapping waves used for imaging.

It is desirable therefore, to provide improved catheter systems that are capable of providing an indication of occlusion while eliminating or significantly reducing exposure of the patient and clinician to imaging waves.

United States Patent specification no. US - A - 2003/176816 discloses a tissue ablation catheter for forming a lesion along a substantially circumferential region of tissue. The catheter includes one or more sensors for monitoring the temperature of the tissue being ablated. The temperature sensors are mounted on the interior or exterior of an expandable member that is affixed to a shaft of the catheter.

United States Patent specification no. US - A - 2006/259023 discloses a method and system for determining contact assessment which includes the steps of positioning a catheter at a tissue treatment site, where the catheter has a proximal end portion and a distal end portion, the proximal end portion defining at least one fluid inlet port and at least one fluid outlet port, an expandable membrane defining a cooling chamber a coolant injection lumen in fluid communication with the at least one fluid inlet port and the cooling chamber, a coolant return lumen in fluid communication with the at least one fluid outlet port and the cooling chamber, the coolant injection tube, the cooling chamber, and the primary coolant return lumen defining a fluid pathway and a temperature sensor located near the coolant return lumen; measuring an internal temperature of the chamber, and modifying the position of the catheter in response to the measured temperature.; The method and system can also use a measured impedance to determine contact assessment.

United States Patent specification no. US - A - 2005/148997 discloses a method whereby pressure is measured on both sides of an occluding member for determining when pressure forces on the occluding member may cause migration of the occluding member. An alarm indicates when the pressure force on the balloon exceeds a predetermined threshold. In another aspect of the invention, a pressure monitor determines when a rate of pressure increase with respect to the fluid volume in the balloon reaches a predetermined threshold when inflating the occluding member. A predetermined amount of fluid is then added to the balloon so that the balloon is not under inflated or over inflated.

US-A-2008/228141 shows an ablation catheter having an elongate shaft, a lumen, an expandable chamber in fluid communication with the lumen (60), and a first and second temperature sensors, one being coupled to the elongate shaft at a location distal to the expandable chamber, the other being coupled to the elongate shaft at a location proximal to the expandable chamber.

### SUMMARY

According to the present invention, there is provided an ablation catheter as specified in claim 1. According to preferred embodiments of the present invention, there is provided an ablation catheter as specified in any of claims 2 - 7.

Aspects, embodiments or examples of the present disclosure which do not fall under the appended claims do not form part of the invention. The present disclosure also relates to methods for measuring a physiologic parameter at one or more regions separated by an expandable chamber. The catheter may be of the type used for performing intracardiac procedures, typically being percutaneously introduced and advanced from the femoral vein in a patient's leg. Alternative methods involve percutaneous introduction into the jugular vein of the patient's neck, or other anatomical entry point that can be used to access the target location within the patient. The method includes treatment of an arrhythmia with a catheter. The catheter may include an expandable chamber for abutting the catheter to a pulmonary vein to occlude blood flow through the vein. For example, differential pressure measurements of the pressure at locations distal and proximal to the expandable chamber may be obtained. In another example, an absolute pressure measurement of pressure at a location distal to the expandable chamber may be obtained. The differential or absolute pressure measurements may be evaluated to guide the placement of the catheter, and in particular, placing the expandable chamber to occlude blood flow in the pulmonary vein. The differential or absolute pressure measurements may be derived continuously during an insertion and treatment procedure to determine appropriate placement of the catheter. Changes in the differential or absolute pressure may be correlated to mechanical occlusion of the pulmonary vein. The present disclosure also relates to systems which include a console for delivery of energy and circulation of a coolant through a catheter. The systems may or may not also include a processing unit for processing signals sensed by sensors positioned on the catheter. The systems may further include a mapping unit that receives information recorded from one or more mapping electrodes positioned on the ablation catheter. The mapping unit may provide electrical activity information to an operator of the system to identify or confirm the location of target tissue.

### BRIEF DESCRIPTION OF THE DRAWINGS

The following drawings are illustrative of particular embodiments of the present disclosure and therefore do not limit the scope of the disclosure. The drawings (not to scale) are intended for use in conjunction with the explanations in the following detailed description, wherein similar elements are designated by identical reference numerals. Moreover, the specific location of the various features is merely exemplary unless noted otherwise.
FIG. 1 illustrates an exemplary ablation catheter of the present disclosure as it would be deployed and used for an ablation procedure.
FIG. 2 illustrates an exemplary system for performing an ablation.
FIGS. 3A and 3B illustrate cross sectional views of catheter as it would be used within the vascular system of a patient.
FIGS. 4A and 4B illustrate signal waveforms of pressure signals indicative of incomplete and complete mechanical occlusion.
FIG. 5 illustrates an ablation system adapted for use with the present disclosure.
FIGS. 6A and 6B illustrate signal waveforms of the pressure distal to the expandable chamber indicative of incomplete and complete mechanical occlusion measured by the single pressure sensing system of FIG. 5.
FIG. 7 illustrates an alternative catheter having a temperature sensor mounted thereon.
FIG. 8 depicts temperature profiles generated from temperature sensor of FIG. 6.
FIG. 9 illustrates an embodiment of a catheter having a flow sensor.
FIG. 10 shows a flow diagram illustrating a process of performing an ablation using a catheter in accordance with embodiments of the present disclosure.

### DETAILED DESCRIPTION

The following description is exemplary in nature and is not intended to limit the scope, applicability, or configuration of the present disclosure in any way. Rather, the description provides practical illustrations for implementing exemplary embodiments of the present disclosure. Moreover, for simplicity and discussion, various figures have been disclosed below in the context of either cryogenic or RF ablation; such disclosure, however, is believed applicable to any catheter-based occlusion and treatment system.

To better understand the environment in which the devices and methods of the present disclosure are used, a general overview of an ablation procedure is believed to be useful. In the catheter-based ablation treatment of cardiac arrhythmias, a specific area of cardiac tissue having aberrant conductive pathways, such as atrial rotors, emitting or conducting erratic electrical impulses, is initially localized.

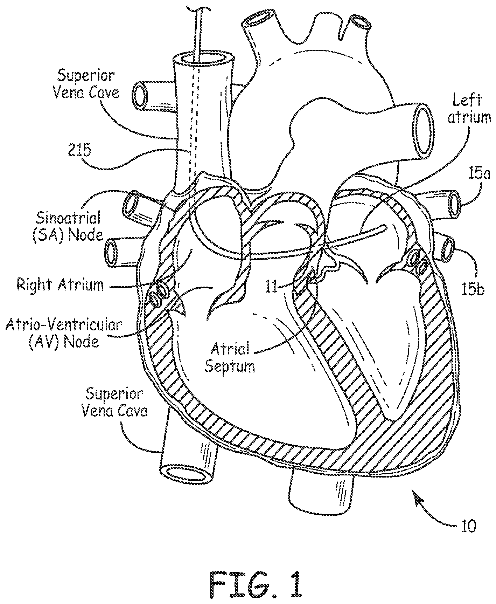

Referring to FIG. 1, the treatment to be accomplished with the devices, systems and methods described in this disclosure is illustrated. FIG. 1 shows a cutaway view of the human heart 10, showing the major structures of the heart 10 including the left and right atria, and the pulmonary veins 15a, 15b. The atrial septum separates the left and right atria. The fossa ovalis 11 is a small depression in the atrial septum that may be used as an access pathway to the left atrium from the right atrium, such as with a transeptal puncture device and transeptal sheath. The fossa ovalis 11 can be punctured, and easily reseals and heals after procedure completion. In a patient suffering from atrial fibrillation, aberrant electrically conducive tissue may be found in the atrial walls, as well as in the pulmonary veins 15a, 15b. Ablation of these areas, referred to as arrhythmogenic foci (also referred to as drivers or rotors), is an effective treatment for atrial fibrillation. Systems, devices and methods of the present disclosure provide means of creating lesions, including lesions to surround the pulmonary vein ostia, and are deployed to identify and ablate the driver and rotor tissue.

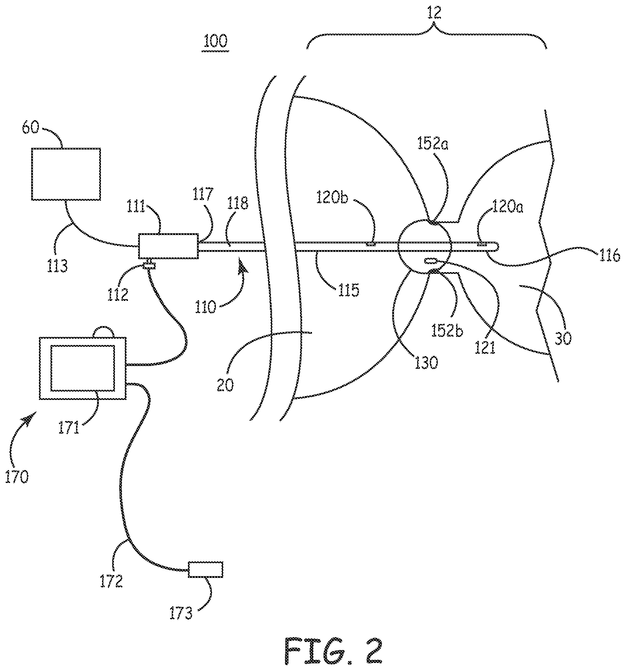

To accomplish this, a catheter (FIG. 2) is inserted into the right atrium, preferably through the inferior vena cava or through the superior vena cava. The catheter is sized for advancement through the patient's vasculature. As an example, which is not intended to be limiting, an exemplary catheter may have a shaft having a diameter ranging from 7-9 Fr, with the shaft length ranging from 100-125 cm and the overall length being in the range of 140-160 cm. The catheter may be passed through transeptal sheath, which may or may not be a deflectable sheath since the catheter preferably includes a deflectable distal portion. When passing into the left atrium, transeptal sheath passes through or penetrates the fossa ovalis 11, such as over guide wire 215 which may have been placed by a transeptal puncture device. The catheter is inserted over guide wire 215 and through transeptal sheath such that its distal end enters the lumen of right superior pulmonary vein 15a, 15b. The catheter carries an ablating element, such as an expandable chamber (FIG. 2) into the left atrium. The expandable chamber is transitioned to expand to a maximal diameter by, for example inflation, such that the expandable chamber is in contact with the walls of the target tissue e.g., pulmonary vein ostia to occlude the vein.

An electrical mapping procedure may be performed to identify or confirm the location of the target cardiac tissue. Next, a treatment medium (e.g., cooling fluid or RF power) provided by a source external to the patient, is provided through the catheter into the ablating element to ablate the neighboring tissue and form a lesion. The created lesions may be segmented and localized. The lesions may be linear or curvilinear, circumferential and partial circumferential, and/or continuous or discontinuous. The lesions created by the ablation catheters are suitable for inhibiting the propagation of inappropriate electrical impulses in the heart 10 for prevention of reentrant arrhythmias. In general, the goal of catheter ablation therapy is to disrupt the electrical pathways in cardiac tissue to stop the emission of and/or prevent the propagation of erratic electric impulses.

FIG. 2 illustrates an exemplary system 100 for performing an ablation. System 100 shows an ablation catheter 110 as it would be used in an ablation procedure of patient 12. Catheter 110 includes elongate catheter body 115 that may suitably be flexible to permit passage through the vascular system of patient 12. The catheter body 115 has a proximal portion 117 that is coupled to a handle 111. Handle 111 may include one or more control knobs 112 for manipulating the catheter body 115 or other components of catheter 110. Handle 111 may be provided with a port (not shown) for receiving a guide wire (not shown) that is passed into one or more lumens 118 of the catheter body 115.

Handle 111 may also include connectors that are coupled directly to an energy source or cryogenic fluid supply/exhaust and control unit or indirectly by way of one or more conduits 113. In the exemplary system, the energy source/fluid supply and exhaust, as well as various control mechanisms for the system are housed in a console 60. However, alternative embodiments may employ a plurality of units to implement the functions of console 60, with each providing a separate function. Console 60 circulates and/or recovers cooling fluid through the catheter body 115 to the patient 12. Additionally, console 60 may provide an exhaust function for the ablation catheter fluid supply.

Catheter body 115 includes one or more lumens for releasing coolant into the expandable chamber 130 responsive to console 60 commands and other control input, such as from the control knobs on handle 111. In the exhaust or recirculation function, console 60 creates a low-pressure environment in the one or more lumens within the catheter body 115. The low-pressure environment draws coolant into lumen 118, away from expandable chamber 130, and towards proximal portion 117. General principles concerning the construction or operation of an exemplary cryogenic system may be found in U.S. 5,281,215 issued to Milder. To the extent not previously discussed the materials and methods of construction may be typical for catheters and guide wires used in coronary arteries.

Catheter body 115 includes a distal portion 116. An expandable chamber 130 is coupled proximate to the distal portion 116. Expandable chamber 130 may also be thermally conductive to facilitate conduction of heat to and from the tissue into a medium that may be carried by the chamber 130. Although expandable chamber 130 is shown as a balloon having a single membrane, it should be understood that any known multi-membrane balloon may suitably be used.

In accordance with aspects of the present disclosure, catheter 110 operates to treat vascular tissue of a patient 12 that is adjacent to the expandable chamber 130 by freezing or through RF energy that may be delivered through electrodes (not shown) mounted on the distal end of catheter 110. To achieve this, catheter body 115 may be navigated through the vascular system to the desired vascular tissue such as a vessel 30. Examples of vessel 30 may include a left pulmonary vein, a right pulmonary vein, ostia, or other blood vessel. During deployment of the catheter 110, expandable chamber 130 may be deflated for ease of steering and passage through the vascular system. Once catheter 110 is adjacent the desired site in vessel 30, expandable chamber 130 may be inflated, as discussed generally in U.S. 6,575,966, issued to Lane et al.. Generally, inflation of expandable chamber 130 will result in radial expansion of expandable chamber 130 to a diameter that is at least as large as that of vessel 30. The expanded expandable chamber 130 may then be advanced to the opening of vessel 30 to achieve contact between expandable chamber 130 and the opening to the interior of vessel 30. When the expandable chamber 130 is properly situated, the blood flow within the vessel 30 will be occluded.

However, the occlusion is predicated upon proper positioning of the expandable chamber 130 to abut with the opening of vessel 30. As previously discussed, proper positioning presents several challenges to the user. These challenges include the difficulty of navigating catheter 110 within the vascular system and the size and nature of the vascular system. The present disclosure utilize one or more physiologic sensors to ascertain the extent of occlusion (and consequently proper location) of the expandable chamber 130.

In fig. 2, a first pressure sensor 120a is coupled to catheter 110 at a location that is anterior or distal to the expandable chamber 130. In use, sensor 120a is in fluid communication with vessel 30 and measures the pressure of the blood flowing within vessel 30. A second pressure sensor 120b may be coupled to catheter 110 at a location that is posterior or proximal to the expandable chamber 130. Pressure sensor 120b may preferably be used in conjunction with sensor 120a to obtain the differential pressure across expandable chamber 130; i.e., the difference between the pressure in the region that is distal to expandable chamber 130 and the pressure in the region that is proximal to expandable chamber 130. The construction and integration of sensors 120a and 120b into the catheter 110 may resemble that disclosed in U.S. Patent 7,231,829 to Michael Schugt.

Sensor 120b operably measures the blood pressure within a body region 20 that is in fluid communication with vessel 30. In an embodiment, region 20 is an atrial chamber adjacent the vessel 30. Accordingly, a computation of the differential pressure in vessel 30 and region 20 can be computed based on the pressure measurements of sensors 120a and 120b. In another embodiment, region 20 may simply be a location that is more distal within vessel 30.

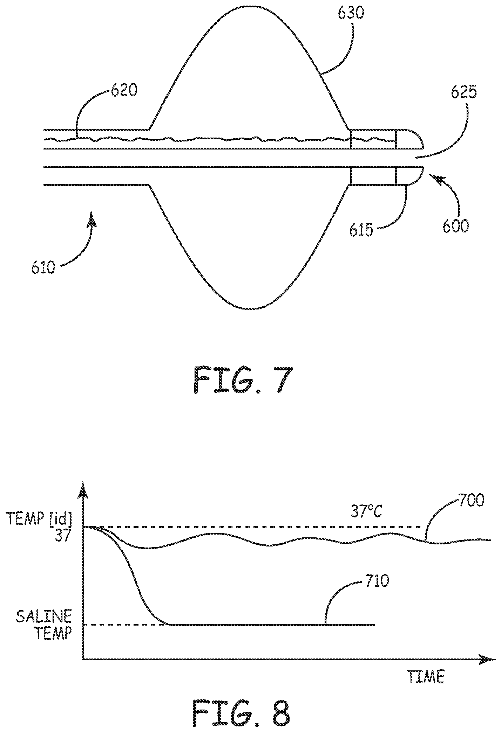

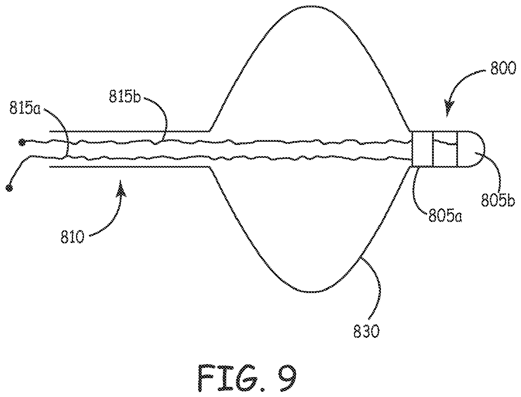

It should be noted that although sensors 120a and 120b have been disclosed in relation to pressure sensors, other forms of sensors may alternatively be used to measure other physiologic and hemodynamic parameters in either or both of region 20 and vessel 30. For example, other sensors such as a temperature sensor (FIG. 7), flow sensor (FIG. 9), an optic sensor, a force sensor, or an electrical sensor or any other suitable sensor known in the art may be substituted.

Catheter 110 may also include a strain gauge 121 that may be coupled to the expandable chamber 130. The strain gauge 121 functions to measure the force exerted on the circumference of the expandable chamber 130. As such, signals obtained by strain gauge 121 can provide an indication of whether the expandable chamber 130 has achieved complete circumferential contact with vessel 30 based on the force (contact) between the circumference of the expandable chamber 130 and the vessel 30 wall.

System 100 may include an output module 170 that is electrically coupled to first and second pressure sensors 120a, 120b for monitoring information sensed by the first and second pressure sensors 120a, 120b. Output module 170 may include signal processing capability comprising a digital signal processor for receiving input signals from the pressure sensors 120a and 120b. The output module 170 may convert the signals to digital form, process those digital signals, and derive an indication of the differential pressure of the blood pressure in region 20 and vessel 30.

The signal processor may correlate the differential pressure computation with a predetermined value. When complete mechanical occlusion has been achieved, the pressure signal waveform in the vessel 30 converts from the pressure signal waveform of region 20 to that of isolated vessel 30. The predetermined value may be obtained by subtracting the signal waveform of the pressure signal in region 20 from the pressure in vessel 30. Computations of the differential pressure measured in vessel 30 and region 20 may be continuously performed and compared against the predetermined value.

The results of differential pressure computation of the pressure in region 20 and vessel 30 may be delivered to a user via display 171. Additionally or alternatively, the raw signals sensed by pressure sensors 120a and 120b may be received by output module 170 and displayed in raw signal waveform on display 171.

In an example, output module 170 may provide an indication to a user, such as a clinician of whether or not occlusion has been achieved or if changes have arisen based on the sensed signals. For example, output module 170 may include a tactile alarm 173 that is worn by the clinician to provide a vibratory signal to the physician when the signals indicate changes in the level of occlusion. Output module 170 may also activate an audible alarm in response to occlusion changes to alert the clinician to indications of possible changes that may require readjustment of the position of catheter 110 or even termination of the process. In other examples, light indicators can be used to instruct the physician about the level of occlusion: for example, a green light indicating occlusion, an orange light indicating partial occlusion and a red light indicating no occlusion.

Catheter 110 may additionally include one or more sensors 152a, 152b for sensing electrical activity of the tissue adjacent the sensors 152a, 12b. Electrical activity signals sensed by sensors 152a, 152b facilitate mapping of the conduction pathways in the tissue. The sensors 152a, 152b may be coupled to output module 170 that performs the mapping procedure to identify or confirm the location of the tissue exhibiting arrhythmia conditions.

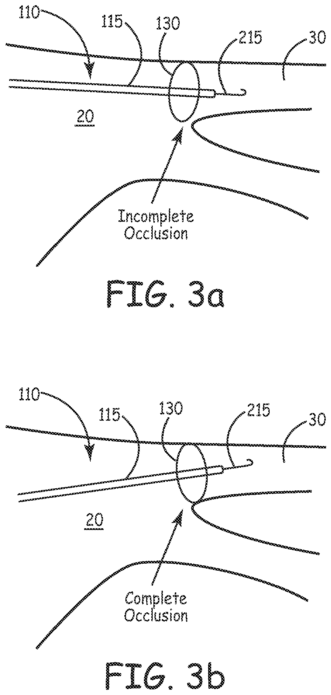

FIGS. 3A and 3B illustrate cross sectional views of catheter 110 as it would be used within the vascular system of a patient. FIG. 3A illustrates catheter 110 with the expandable chamber 130 radially expanded, e.g., by inflation. As further shown in the embodiment, a guide wire 215 is used for over-the-wire insertion of catheter 110 through the vascular system to vessel 30. It should be noted that the lumen in which the guide wire 215 resides is filled with a fluid such as saline, contrast or body fluid. This configuration allows use of the catheter 110 by insertion through region 20, such as a cardiac chamber to abut vessel 30 exiting the chamber. The expandable chamber 130 is shown positioned to near a desired site at vessel 30. In this orientation, however, expandable chamber 130 will not completely occlude or block the flow of blood from region 20 through vessel 30 because of the interruptions in the circumferential contact with the opening to the interior of vessel 30 at the target site.

Turning now to FIG. 3B, expandable chamber 130 is shown positioned within vessel 30 in accordance with principles of the present disclosure. Catheter 110 is navigated through the vascular system and with the aid of the measured differential pressure measurements, as discussed in FIG. 2, expandable chamber 130 may be positioned such that its external circumferential surface is in an uninterrupted contact with the opening to the interior of vessel 30. The continuous circumferential contact between the opening of vessel 30 and expandable chamber 130 enables complete occlusion of blood flow within vessel 30.

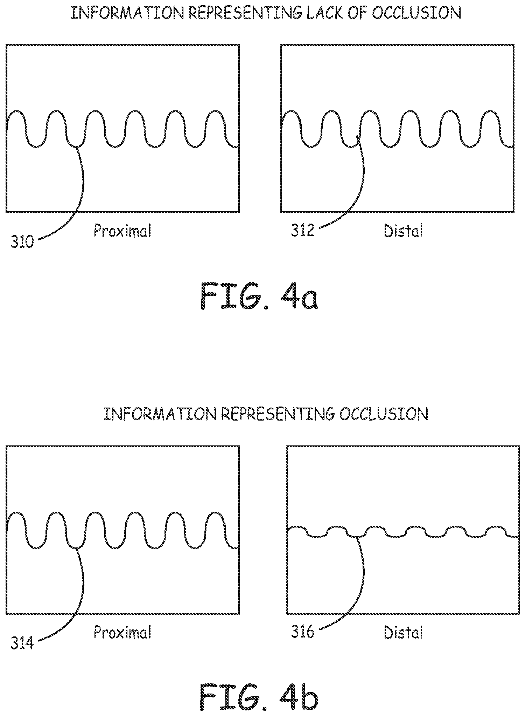

FIGS. 4A and 4B illustrate signal waveforms 310, 312, 314, and 316 of pressure signals indicative of incomplete and complete occlusion. In accordance with the present disclosure, the pressure sensors 120a and 120b may be utilized to measure the blood pressure within vessel 30 and region 20, respectively, to determine whether or not the expandable chamber 130 has achieved complete occlusion. The signal waveforms 310, 312, 314, 316 illustrated in FIGS. 3A and 3B may be viewed in relation to FIG. 4A and FIG. 4B as described in detail below.

In FIG. 4A, correlating to FIG. 3A, the signal waveform 310 corresponds to the pressure of blood flow within region 20 whereas the signal waveform 312 corresponds to the blood flow within vessel 30. FIG. 4B, correlates to FIG. 3B where there is a complete occlusion of vessel 30. As depicted in FIGS. 4A and 4B, the signal waveforms 310 and 312 at the proximal and distal location will have identical or substantially identical waveforms for a non-occluded vessel 30. In contrast, the signal waveforms 314 and 316 at the proximal and distal location will differ when the vessel 30 is occluded.

In an alternative example, the signal waveforms of the pressure measurement in region 20 and vessel 30 may be processed by output module 170 to provide a visual representation of a composite waveform that aggregates the signal waveforms of both region 20 and vessel 30. Alternatively, output module 170 may perform signal processing of the sensed signals to provide other parameters, including but not limited to text, numerical or graphical representations of the differential pressure.

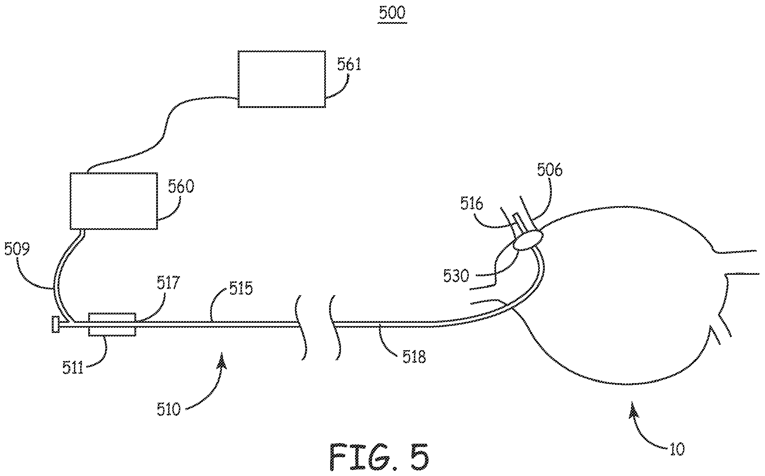

FIG. 5 illustrates a catheter-based ablation system 500 adapted for use in accordance with an alternative example of the present disclosure. An ablation catheter 510 is illustrated as it would be used, in one example, in heart 10 to achieve occlusion of a pulmonary vein 506. A body 515 of the catheter 510 has a proximal portion 517 and a distal portion 516 with a lumen 518 therethrough. An expandable chamber 530 is coupled at the distal portion 516. Expandable chamber 530 may be in fluid communication with lumen 518 to facilitate selective expansion of the expandable chamber 530. The proximal portion 517 includes a handle 511 which may include one or more control knobs and an orifice in communication with the lumen 518.

Catheter 510 may be coupled to a console 560 through a tubular connector 509. The connector 509 may be in fluid communication with the lumen 518 to permit pressure wave transmission, via a fluid, from pulmonary vein 506 to connector 509. Console 560 may include control electronics including, but not limited to, a pressure gauge and signal processing circuitry.

In an example, console 560 processes the mechanical pressure exerted on the distal opening of catheter 510 and transmitted through the fluid in lumen 518. To achieve this, fluid such as saline is supplied into the lumen 518 to substantially fill up the lumen 518. As such, when the distal portion 516 of catheter 510 is located within or adjacent pulmonary vein 506, blood flow within the pulmonary vein 506 comes into contact with the distal opening of catheter 510. Occlusion of the pulmonary vein 506 by the expandable chamber 530 may be determined based on the mechanical pressure exerted by this blood flow.

In accordance with principles of this disclosure, the blood flow in the pulmonary vein 506 causes a mechanical deflection of the fluid at the distal opening of lumen 518. The mechanical deflection corresponds to the mechanical pressure exerted by the blood flowing adjacent to the distal portion 516. The mechanical deflection of the fluid at the tip of distal portion 516 is transmitted to the proximal portion of catheter 510. This deflection of the fluid in lumen 518 may be sensed and processed by console 560 which is in fluid communication with the lumen 518. In alternative examples, a separate pressure gauge/sensor may be coupled to catheter 510 for determination of the mechanical pressure exerted on the distal portion 516. As such, system 500 correlates the mechanical occlusion at a location distal to the expandable chamber 530 to the pressure exerted on the distal portion 516.

The mechanical pressure signal corresponding to the pressure exerted on the catheter body 516 may be processed and a result of the processing delivered to the user via display 561. The result displayed may be a graphical, text, numerical, pictorial, or any other suitable indication of the determination of occlusion. Additionally or alternatively, the sensed raw signal waveform may be displayed directly on the display 561.

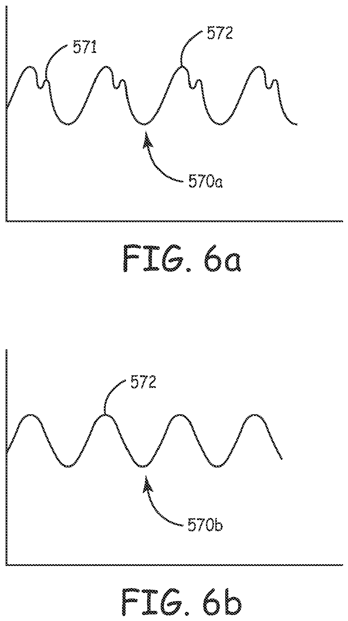

FIGS. 6A, 6B illustrate pressure waveforms of mechanical pressure exerted on the catheter body 515 of FIG. 5. These raw signal waveforms may be provided to the user on display 561. The illustration in FIG. 6A depicts an exemplary pressure waveform 570a of pulmonary vein 506 prior to occlusion by the expandable chamber 530. The pressure in the atrial chamber adjacent the pulmonary vein 506 fluctuates based on the changes in the cardiac phase. Similarly, the pressure within the first three to five centimeters in the pulmonary vein 506 substantially fluctuates in a similar pattern to the pressure in the adjoining atrial chamber. Therefore, in a non-occluded or partially occluded case, the pressure signal sensed in the pulmonary vein 506 would contain a component of the pressure in the adjoining atrial chamber and the ventricular pressure. Pressure waveform 570a includes an atrial A pressure component 571 corresponding to atrial mechanical contraction and a ventricular V pressure component 572. The presence of both the atrial A component 571 and ventricular V component 572 in the pressure waveform 570a monitored in the pulmonary vein 506 indicates that the pulmonary vein 506 is not occluded or at least is only partially occluded.

FIG. 6B depicts pressure waveform 570b of a completely occluded pulmonary vein 506. Pressure waveform 570b includes only the ventricular V pressure component 572 with complete disappearance of the atrial A pressure component 571. The conversion of the monitored pressure waveform 570a (FIG. 6A) to the pressure waveform 570b, indicates a complete occlusion by the expandable chamber 530 and hence occlusion of blood flow in the pulmonary vein 506.

It should be noted that for the cryogenic based ablation, the absolute pressure monitoring illustrated in the embodiment of FIG. 5 may be inhibited by the flow of the cooling fluid. This is because the cooling fluid flowing in the lumen 518 may be cooled to below a freezing temperature which may prevent the fluid transmission of mechanical deflections indicative of the pressure. Accordingly, a pressure sensor may additionally be coupled to the catheter 510 distal to the expandable chamber 530.

FIG. 7 illustrates a catheter 610 having a temperature sensor 600 mounted thereon. Temperature sensor 600 includes a conductive element 615 that is coupled to an electrically conductive wire 620 for electrical coupling of the conductive element 615 to electronic circuitry (not shown). The electronic circuitry cooperates with the temperature sensor 600 to sense the temperature of the tissue/environment surrounding temperature sensor 600. The temperature measurements may be used to provide information regarding occlusion. A temperature gradient may be created at the location of the tissue/environment surrounding the temperature sensor 600 by introducing saline through a lumen 625. The saline may be at a higher or lower temperature than the patient's blood/body temperature, provided there is a temperature difference between the surrounding blood and/or tissue/environment and the saline. For example, cold saline at a temperature in the range of about twenty degrees Celsius to thirty-five degrees Celsius may be used.

In an example, using in-vivo or in-vitro modeling, appropriate temperature profiles as measured by the temperature sensor 600 can be obtained for the case of occlusion, partial occlusion or no occlusion. These profiles can be incorporated into the console (not shown) and compared with real-time measurements to determine occlusion. In an example, a large temperature change, e.g., greater than five degrees Celsius, as measured by the temperature sensor 600 may be associated with complete occlusion whereas a temperature change of two degrees Celsius or less may be associated with no occlusion. Temperature differences between two and five degrees Celsius may be designated as corresponding to partial occlusion. However, one skilled in the art will appreciate that the temperature variances noted above are merely illustrative and as a matter of routine use, temperature profiles tailored to specific classes of patients can easily be obtained.

In an alternative example, conductive element 615 may serve a dual function, i.e., as a sensor and an electrode. As such, conductive element 615 may be used for electrical mapping or may be used to provide information about tip location during navigation.

FIG. 8 depicts temperature profiles 700, 710 generated from temperature sensor 600. In use, catheter 610 is advanced into a desired chamber and an expandable chamber 630 is positioned adjacent the target tissue. Saline is injected into lumen 625 of the catheter 610 and exits through a distal opening of catheter 610. As discussed above, the saline may be at a higher or lower temperature than the patient's blood/body temperature. In this example, the saline is at a lower temperature. The illustration of temperature profile 700 indicates an occluded vessel. If the desired vessel is occluded, the saline will displace or mix with the stationary blood, which has a known temperature-typically, about thirty-seven degrees Celsius-creating a decrease in the temperature measured by the temperature sensor 600. In contrast, temperature profile 710 indicates a vessel that has not been occluded. If the vessel is not occluded, the saline will be entrained by the blood flowing past the expandable chamber 630, resulting in no or insignificant change in the temperature profile around the conductive element 615.

FIG. 9 illustrates a catheter 810 having a flow sensor 800. Flow sensor 800 has a proximal conductive element 805a and a distal conductive element 805b. Conductive element 805a is coupled to electrically conductive wire 815a while conductive element 805b is coupled to electrically conductive wire 815b. Each of wires 815a, 815b is electrically coupled to electronic circuitry (not shown) for obtaining output signals from the conductive elements 805a, 805b. In an embodiment, flow sensor 800 is a calorimetric flow measuring device such as that disclosed in U.S. 6,539,791 issued to Weber and U.S. 5,390,541 issued to Feller. Catheter 810 also includes an expandable chamber 830 which is constructed in accordance with the description of expandable chamber 130 (FIG. 2). In accordance with an exemplary method of use, catheter 810 is navigated to the desired chamber and expandable chamber 830 placed adjacent the target vessel to occlude blood flow as generally described above.

In accordance with the present disclosure, operation of flow sensor 800 is characterized as follows: if there is no flow and the fluid is stationary (as in the case of an occluded vessel), there will be a constant temperature difference between the proximal conductive element 805a and the distal conductive element 805b. The temperature of the distal element 805b will correspond generally to the temperature of the heat source and the temperature of the proximal element 805a will correspond generally to the temperature of the stationary blood. On the other hand, if fluid flow is present across the two elements 805a, 805b (as in the case of a partially or non-occluded vessel), the fluid will draw heat away from the heated element 805b and the temperature difference between the two elements 805a, 805b will be smaller or the same. The rate of cooling of element 805b is proportional to flow rate.

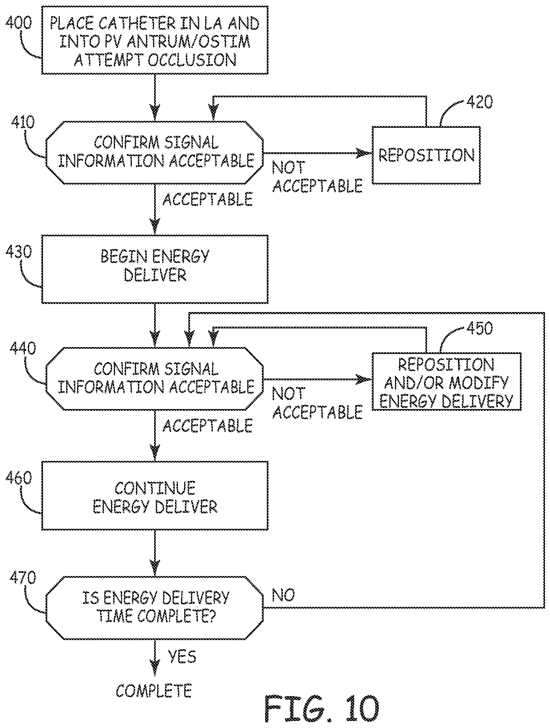

FIG. 10 shows a flow diagram illustrating a process of performing an ablation using the catheters of the present disclosure. The process may be initiated with the placement 400 of any one of the catheters (110, 510, 610, or 810) of the present disclosure into a region 20, such as the left atrium with the corresponding expandable chamber (130, 530, 630, or 830) positioned to abut a vessel 30 such as a pulmonary vein or ostium. The expandable chamber may be expanded to a desired size prior to contact with vessel 30. A physiologic parameter may be measured to guide the positioning of expandable chamber in vessel 30 as described above in reference to the various embodiments of the catheters. In the case of catheter 110, the physiologic parameter measured is the differential pressure. In the case of catheter 510, the physiologic parameter measured is the absolute pressure. In the case of catheter 610, the physiologic parameter measured is temperature. In the case of the catheter 810, the physiologic parameter measured is flow. For ease of description, the ensuing description of the various steps in the process will be described in relation to catheter 110 unless noted otherwise.

At step 410, the physiologic parameter is evaluated to confirm whether the signal information is indicative of an appropriate placement of the expandable chamber 130 that denotes that complete occlusion has been achieved. The evaluation may be performed on the raw sensed signal or information derived from processing the sensed signal. In either event, if the sensed signal is not acceptable, the catheter 110 may be manipulated 420 with the aid of the sensed signals to abut the vessel 30 and achieve complete occlusion. The manipulation may include torquing, advancing, retracting, repositioning, inflating, or deflating the expandable chamber 130.

The tissue ablation may be initiated at step 430 upon confirmation that the expandable chamber 130 has achieved complete occlusion. For example, in a cryogenic ablation, a cooling fluid may be circulated through catheter 110 by console 60 into expandable chamber 130. The energy transfer phenomenon is utilized to create a net transfer of heat from the target tissue on vessel 30 into the cooling fluid. Because of the circulation of cooling fluid by console 60, energy is extracted from the target tissue by the cooling fluid. The rate and magnitude of energy transfer can be controlled by the controls on handle 111. A count of a predetermined duration for circulating the cooling fluid may also be initiated. In the case of RF ablation, RF energy may be delivered from console 60 via electrodes to form the lesions on the desired tissue.

Step 440 denotes an optional process of continual, intermittent or on-demand monitoring of the physiologic parameter to determine the occlusion of the vessel 30. The optional monitoring of the physiologic parameter at step 440 may facilitate the determination of occlusion of vessel 30 during the ablation procedure. Determining whether vessel 30 is continuously occluded during the tissue ablation may be useful because blood flow during the procedure may be undesirable. This is because the blood flow may inhibit effective cooling of the target tissue of vessel 30 due to the presence of heat in the flowing blood or in the case of RF energy, the blood may lower the temperature of the delivered energy. If the monitored physiologic parameter at step 440 indicates that vessel 30 is properly occluded, the ablation is continued 460.

At step 470, the process determines whether the predetermined duration for delivery of the cooling fluid has elapsed. If the duration has not elapsed, the system will maintain the monitoring of the physiologic parameter at step 440 to determine whether the vessel 30 is still completely occluded.

In an alternative example, the monitored physiologic parameter at step 440 may indicate the recurrence of blood flow. Responsive to the indication of recurrence of blood flow at 440, various adjustments 450 may be performed. For instance, the catheter 110 may be repositioned and/or the temperature or flow rate of the cooling fluid may be modified.

In other aspects of the disclosure, the physiologic parameter measurement may be utilized in conjunction with an imaging procedure. For example, imaging may be performed through fluoroscopy to verify proper placement and complete occlusion of the blood vessel 30. However, the physiologic parameter measurements may significantly reduce the imaging time thereby reducing exposure of the patient and clinician to radiation waves of the imaging procedure.

The present disclosure may also be used in combination with devices that deliver one or more forms and types of energy for ablation therapy including but not limited to: sound energy such as acoustic energy and ultrasound energy; electromagnetic energy such as electrical, magnetic, microwave and radiofrequency energies; thermal energy such as heat energy; chemical energy such as energy generated by delivery of a drug; laser or light energy such as infrared and visible light energies; mechanical and physical energy; radiation; and combinations thereof.

In another alternative example, the patient's phrenic nerve may be paced prior to measurement of the physiologic parameter. Methods and devices for phrenic nerve stimulation are known in the art and include U.S. Patent 7,225,019, issued to Jahns, et al.. The invention is defined by the appended claims.

## Claims

1. An ablation catheter comprising:
an elongate shaft (115) with a proximal end (117) and a distal end (116) and a lumen (118) disposed between the proximal end (117) and the distal end (116);
an expandable chamber (130) in fluid communication with the lumen (118) coupled proximate to the distal end (116);
a first physiologic sensor (120a) coupled to the elongate shaft (115), the first physiologic sensor (120a) being coupled at a location that is distal to the expandable chamber (130) on the elongate shaft (115);
a second physiologic sensor (120b) coupled to the elongate shaft (115), the second physiologic sensor (120b) being coupled at a location that is distal to the expandable chamber (130) on the elongate shaft (115); and
the first (120a) and second (120b) physiologic sensors being operable to measure a parameter indicative of an extent of occlusion in a blood vessel when the expandable chamber (130) is inserted within the blood vessel and inflated; wherein the first (120a) and second (120b) physiologic sensors comprise a calorimetric flow sensor, wherein the first (120a) and second (120b) physiologic sensors provide a measurement of the temperature variation between the first physiologic sensor (120a) and the second physiologic sensor (120b) for derivation of the flow of a medium.

2. The catheter of claim 1, wherein the expandable chamber (130) comprises a tissue contact surface, and the first (120a) and second (120b) physiologic sensors are configured to detect heat flow proximate the tissue contact surface.

3. The catheter of claim 1, wherein the expandable chamber (130) comprises a fluidmedium inflatable balloon.

4. The catheter of claim 1, further comprising a handle (111) coupled to the elongate shaft (115), wherein the handle (111) includes a control knob (112) for manipulating the elongate shaft (115).

5. The catheter of claim 1, wherein the expandable chamber (130) comprises at least one portion that is compliant.

6. The catheter of claim 1, wherein the expandable chamber (130) comprises at least one portion that is non-compliant.

7. The catheter of claim 1, wherein the expandable chamber (130) comprises a first portion configured to abut a pulmonary vein.

## Patentansprüche

1. Ablationskatheter, der Folgendes umfasst:
einen langgestreckten Schaft (115) mit einem proximalen Ende (117) und einem distalen Ende (116) und einem Lumen (118), das zwischen dem proximalen Ende (117) und dem distalen Ende (116) angeordnet ist;
eine expandierbare Kammer (130) in Fluidverbindung mit dem Lumen (118), die nah bei dem distalen Ende (116) gekoppelt ist;
einen ersten physiologischen Sensor (120a), der an den langgestreckten Schaft (115) gekoppelt ist, wobei der erste physiologische Sensor (120a) an einer Stelle gekoppelt ist, die distal zu der expandierbaren Kammer (130) an dem langgestreckten Schaft (115) ist;
einen zweiten physiologischen Sensor (120b), der an den langgestreckten Schaft (115) gekoppelt ist, wobei der zweite physiologische Sensor (120b) an einer Stelle gekoppelt ist, die distal zu der expandierbaren Kammer (130) an dem langgestreckten Schaft (115) ist; und
wobei der erste (120a) und der zweite (120b) physiologische Sensor wirksam sind, um einen Parameter zu messen, der ein Ausmaß von Okklusion in einem Blutgefäß angibt, wenn die expandierbare Kammer (130) in das Blutgefäß eingeführt und aufgeblasen ist; wobei der erste (120a) und der zweite (120b) physiologische Sensor einen kalorimetrischen Durchflusssensor umfassen, wobei der erste (120a) und der zweite (120b) physiologische Sensor eine Messung der Temperaturschwankung zwischen dem ersten physiologischen Sensor (120a) und dem zweiten physiologischen Sensor (120b) zum Herleiten des Durchflusses eines Mediums bereitstellen.

2. Katheter nach Anspruch 1, wobei die expandierbare Kammer (130) eine Gewebekontaktfläche umfasst, und der erste (120a) und der zweite (120b) physiologische Sensor dazu konfiguriert sind, Wärmefluss nah bei der Gewebekontaktfläche zu erkennen.

3. Katheter nach Anspruch 1, wobei die expandierbare Kammer (130) einen mit einem Fluidmedium aufblasbaren Ballon umfasst.

4. Katheter nach Anspruch 1, weiter umfassend einen Griff (111), der an den langgestreckten Schaft (115) gekoppelt ist, wobei der Griff (111) einen Bedienungsknopf (112) zum Manipulieren des langgestreckten Schafts (115) umfasst.

5. Katheter nach Anspruch 1, wobei die expandierbare Kammer (130) mindestens einen Abschnitt umfasst, der nachgiebig ist.

6. Katheter nach Anspruch 1, wobei die expandierbare Kammer (130) mindestens einen Abschnitt umfasst, der nicht nachgiebig ist.

7. Katheter nach Anspruch 1, wobei die expandierbare Kammer (130) einen ersten Abschnitt umfasst, der dazu konfiguriert ist, an einer Pulmonalvene anzuliegen.

## Revendications

1. Cathéter d'ablation comportant :
une tige allongée (115) avec une extrémité proximale (117) et une extrémité distale (116) et une lumière (118) disposée entre l'extrémité proximale (117) et l'extrémité distale (116) ;
une chambre extensible (130) en communication fluidique avec la lumière (118) accouplée à proximité de l'extrémité distale (116) ;
un premier capteur physiologique (120a) accouplé à la tige allongée (115), le premier capteur physiologique (120a) étant accouplé au niveau d'un emplacement qui est distal par rapport à la chambre extensible (130) sur la tige allongée (115) ;
un deuxième capteur physiologique (120b) accouplé à la tige allongée (115), le deuxième capteur physiologique (120b) étant accouplé au niveau d'un emplacement qui est distal par rapport à la chambre extensible (130) sur la tige allongée (115) ; et
les premier (120a) et deuxième (120b) capteurs physiologiques servant à mesurer un paramètre indiquant l'étendue d'une occlusion dans un vaisseau sanguin quand la chambre extensible (130) est insérée dans le vaisseau sanguin et gonflée ; dans lequel les premier (120a) et deuxième (120b) capteurs physiologiques comportent une capteur de flux calorimétrique, dans lequel les premier (120a) et deuxième (120b) capteurs physiologiques fournissent une mesure de la variation de température entre le premier capteur physiologique (120a) et le deuxième capteur physiologique (120b) à des fins de dérivation du flux d'un milieu.

2. Cathéter selon la revendication 1, dans lequel la chambre extensible (130) comporte une surface en contact avec le tissu, et les premier (120a) et deuxième (120b) capteurs physiologiques sont configurés pour détecter le flux thermique à proximité de la surface en contact avec le tissu.

3. Cathéter selon la revendication 1, dans lequel la chambre extensible (130) comporte un ballonnet gonflable à milieu fluide.

4. Cathéter selon la revendication 1, comportant par ailleurs une poignée (111) accouplée à la tige allongée (115), dans lequel la poignée (111) comprend un bouton de commande (112) servant à manipuler la tige allongée (115).

5. Cathéter selon la revendication 1, dans lequel la chambre extensible (130) comporte au moins une partie qui est élastique.

6. Cathéter selon la revendication 1, dans lequel la chambre extensible (130) comporte au moins une partie qui est non élastique.

7. Cathéter selon la revendication 1, dans lequel la chambre extensible (130) comporte une première partie configurée pour venir en butée contre une veine pulmonaire.
